Europäisches Patentamt

European Patent Office

Office européen des brevets

(11) Veröffentlichungsnummer: **0 276 806 B1**

# EUROPÄISCHE PATENTSCHRIFT

(45) Veröffentlichungstag der Patentschrift: **31.03.93**

(21) Anmeldenummer: **88101059.9**

(22) Anmeldetag: **26.01.88**

(51) Int. Cl.⁵: **C12N 9/26**, C12N 9/44

(54) Thermostabile Amylasen und Pullulanasen aus 2 anaeroben Mikroorganismen.

(30) Priorität: **29.01.87 DE 3702626**

(43) Veröffentlichungstag der Anmeldung:
**03.08.88 Patentblatt 88/31**

(45) Bekanntmachung des Hinweises auf die
Patenterteilung:
**31.03.93 Patentblatt 93/13**

(84) Benannte Vertragsstaaten:
**AT BE CH DE ES FR GB IT LI NL**

(56) Entgegenhaltungen:
EP-A- 0 131 253     EP-A- 0 180 952
EP-A- 0 184 019     EP-A- 0 258 050
EP-A- 0 268 193     WO-A-86/01831

APPLIED AND ENVIRONMENTAL MICROBIO-
LOGY, Band 53, Nr. 7, Juli 1987, Seiten
1668-1673, American Society for Microbiology; G. ANTRANIKIAN et al.: "Production of
thermostable alpa-amylase, pullulanase, and
alpha-glucosidase in continuous culture by
a new Clostridium isolate"

APPLIED MICROBIOL BIOTECHNOL, Band 27,
November 1987, Seiten 192-198, Springer-
Verlag; R. KOCH et al.: "Highly active and
thermostable amylases and pullulanases

from various anaerobic thermophiles"

(73) Patentinhaber: **Kali-Chemie Aktiengesellschaft
Postfach 220, Hans-Böckler-Allee 20
W-3000 Hannover 1(DE)**

(72) Erfinder: **Antranikian, Garabed, Dr.
Grisebachstrasse 8
W-3400 Göttingen(DE)**
Erfinder: **Gottschalk, Gerhard, Prof. Dr.
Grisebachstrasse 8
W-3400 Göttingen(DE)**
Erfinder: **Koch, Reinhard, Dr.
Grisebachstrasse 8
W-3400 Göttingen(DE)**

(74) Vertreter: **Lauer, Dieter, Dr.
c/o Kali-Chemie AG Postfach 220 Hans-
Böckler-Allee 20
W-3000 Hannover 1 (DE)**

**Beschreibung**

Die Erfindung betrifft thermostabile Amylasen und Pullulanasen aus Clostridium thermosaccharolyticum DSM 572 und Thermoanaerobacter ethanolicus DSM 2246.

Als Pullulanase wurde ein Enzyme definiert, das in der Lage ist, Pullulan zu hydrolysieren. Als Amylase wurde ein Enzyme definiert, das in der Lage ist, Stärke zu hydrolysieren.

Die Bildung von amylolytischen, d.h. Stärke abbauenden Enzymen ist bekannt. So wird nach WO-A-86.01 831 Clostridium thermohydrosulfuricum ATCC 33 223 ansatzweise auf einem Kulturmedium mit einem Gehalt an 0,5 % löslicher Stärke in Batchkultur kultiviert, wobei zwar Amylase, Pullulanase und Glucoamylase gebildet, jedoch nicht in das Kulturmedium ausgeschieden werden; vgl. auch Appl. Envir. Microbiol., 49 (1985) 1168-1173. Nach WO-A-86.01 832 wird Clostridium thermosulfurogenes ATCC 33 743 = DSM 2229 ansatzweise in einem Kulturmedium mit 0,5 % löslicher Stärke kultiviert, wobei Amylase, Pullulanase und Glucoamylase als amylolytische Enzyme gebildet werden, jedoch nur das zuerst genannte Enzym in das Kulturmedium austritt.

Aufgabe der Erfindung ist es, thermostabile amylolytische Enzyme bereitzustellen, die bei der Kultivierung von thermophilen, die genannten Enzyme bildenden Bakterien in hohem Maß in das Kulturmedium ausgeschieden werden und damit leicht isoliert werden können.

Clostridium thermosaccharolyticum DSM 572 und Thermoanaerobacter ethanolicus DSM 2246 produzieren thermostabile Amylase und Pullulanase. Bei Stärke als Substrat werden in kontinuierlicher Kultur über 90 % beider Enzyme ins Medium ausgeschieden.

Die in kontinuierlicher Kultur erreichten maximalen Aktivitäten beider Enzyme waren folgende:

1) Thermoanaerobacter ethanolicus DSM 2246

Pullulanase:   12300 U/l extrazellulär, 97 % des Enzyms im Überstand
            300 U/l zellgebunden, 3 % des Enzyms zellgebunden
Amylase:   4670 U/l extrazellulär, 92 % des Enzyms im Überstand
            390 U/l zellgebunden, 8 % des Enzyms zellgebunden
Die Bedingungen der optimalen Enzymproduktion im Fermenter waren:
- pH 5,6
- 1 % Stärke im Medium
- Durchflußrate 0,039/h
- Temperatur: 65 °C

2) Clostridium thermosaccharolyticum DSM 572

Pullulanase:   3400 U/l extrazellulär, 92 % des Enzyms im Überstand
            300 U/l zellgebunden, 8 % des Enzyms zellgebunden

Amylase:   1960 U/l extrazellulär, 96 % des Enzyms im Überstand
            80 U/l zellgebunden, 4 % des Enzmys zellgebunden
Die Bedingungen der optimalen Enzymproduktion im Fermenter waren:
- pH 6,0
- 1 % Stärke im Medium
- Durchflußrate 0,063/h
- Temperatur: 60 °C
Nachstehend wird die Erfindung mit Abbildungen näher erläutert. Es zeigen:
Abbildung 1   die Abhängigkeit der Enzymaktivität von der Temperatur für T. ethanolicus DSM 2246;
Abbildung 2   die Abhängigkeit der Enzymaktivität vom pH-Wert für T. ethanolicus DSM 2246;
Abbildung 3   die Abhängigkeit der Amylaseaktivität von der Zeit für T. ethanolicus DSM 2246;
Abbildung 4   die Abhängigkeit der Pullulanaseaktivität von der Zeit für T. ethanolicus DSM 2246;
Abbildung 5   die Abhängigkeit der Pullulanaseaktivitäten von der Zeit für C. thermosaccharolyticum DSM 572 und T. ethanolicus DSM 2246;
Abbildung 6   die Abhängigkeit der Enzymaktivität von der Temperatur für C. thermosaccharolyticum DSM 572;
Abbildung 7   die Abhängigkeit der Enzymaktivität vom pH-Wert für C. thermosaccharolyticum DSM 572;
Abbildung 8   die Abhängigkeit der Amylaseaktivität von der Zeit für C. thermosaccharolyticum DSM

2

572; und

Abbildung 9    die Abhängigkeit der Pullulanaseaktivität von der Zeit für C. thermosaccharolyticum DSM 572.

Charakterisierung der Enzyme

1) Thermoanaerobacter ethanolicus DSM 2246

Temperaturoptimum von Amylase und Pullulanase

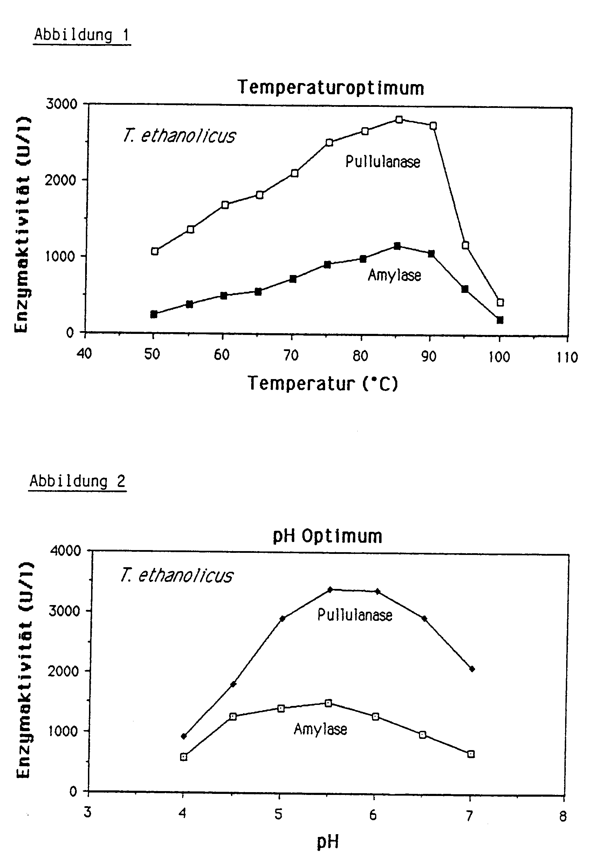

Die Amylase ist optimal aktiv zwischen 75 °C und 90 °C, insbesondere zwischen 80 °C und 90 °C. Die Pullulanase ist ebenfalls optimal aktiv zwischen 75 °C und 90 °C, insbesondere zwischen 80 °C und 90 °C. Beide Enzyme sind aktiv bei Temperaturen zwischen 50 °C und 100 °C, insbesonders zwischen 70 °C und 95 °C, vorzugsweise zwischen 75 °C und 90 °C und besonders bevorzugt zwischen 85 °C und 90 °C. Siehe dazu Abbildung 1.

pH-Optimum von Amylase und Pullulanase

Die Amylase ist optimal aktiv zwischen pH 5,0 und 6,0. Die Pullulanase ist optimal aktiv zwischen pH 5,0 und 6,5, insbesondere zwischen pH 5,5 und 6,0. Beide Enzyme sind aktiv bei pH-Werten zwischen 4 und 7, insbesonders zwischen 4,5 und 6,5, vorzugsweise zwischen pH 5,0 und 6,2 und besonders bevorzugt zwischen pH 5,5 und 6,0. Siehe dazu Abbildung 2.

Beide Enzyme sind unter den gleichen Bedingungen (pH-Wert und Temperatur) katalytisch aktiv, so daß sie in einem Schritt zur Stärkehydrolyse eingesetzt werden können. Hierfür werden keine Metallionen benötigt. Die Experimente wurden unter aeroben Bedingungen und ohne Substratzugabe durchgeführt.

Enzymaktivität in Gegenwart von Metallen

In Gegenwart von Zink und Kupfer fällt die Aktivität von Amylase und Pullulanase ab. Die übrigen Metalle hatten keinen großen Einfluß auf die Enzymaktivität (Tabelle 1).

Tabelle 1

Abhängigkeit der Enzymaktivität von Metallionen

| | Clostridium thermosaccharolyticum | | Thermoanaerobacter ethanolicus | |
|---|---|---|---|---|
| Metall | Amylase | Pullulanase | Amylase | Pullulanase |
| 0 mM | 100% | 100% | 100% | 100% |
| $CoCl_2 x 6H_2O$ | | | | |
| 0,4 mM | 124% | 94% | 114% | 99% |
| 1,6 mM | 89% | 93% | 106% | 99% |
| 4,0 mM | 99% | 50% | 88% | 99% |
| $Na_2MoO_4 x 2H_2O$ | | | | |
| 0,4 mM | 104% | 85% | 93% | 100% |
| 1,6 mM | 86% | 92% | 92% | 95% |
| 4,0 mM | 91% | 93% | 91% | 99% |
| $ZnSO_4 x 7H_2O$ | | | | |
| 0,4 mM | 52% | 29% | 34% | 62% |
| 1,6 mM | 14% | 3% | 16% | 16% |
| 4,0 mM | 2% | 0% | 8% | 14% |
| $NiCl_2 x 6H_2O$ | | | | |
| 0,4 mM | 92% | 80% | 89% | 76% |
| 1,6 mM | 60% | 33% | 71% | 79% |
| 4,0 mM | 33% | 32% | 54% | 71% |
| $MnCl_2 x 4H_2O$ | | | | |
| 0,4 mM | 99% | 109% | 98% | 112% |
| 1,6 mM | 100% | 96% | 76% | 110% |
| 4,0 mM | n.d. | n.d. | 57% | 81% |
| $CaCl_2$ | | | | |
| 0,4 mM | 124% | 175% | 117% | 107% |
| 1,6 mM | 109% | 175% | 113% | 105% |
| 4,0 mM | 113% | 163% | 91% | 113% |
| $CuSO_4 x 5H_2O$ | | | | |
| 0,4 mM | 5% | 0% | 4% | 6% |
| 1,6 mM | 0% | 0% | 0% | 1% |
| 4,0 mM | 0% | 0% | 0% | 1% |
| $MgSO_4 x 7H_2O$ | | | | |
| 0,4 mM | 93% | 78% | 87% | 82% |
| 1,6 mM | 80% | 82% | 76% | 78% |
| 4,0 mM | 79% | 61% | 81% | 69% |

n.d. = nicht ermittelt

Enzymaktivität in Gegenwart von Cyclodextrinen

Amylase und Pullulanase werden durch α-Cyclodextrin nicht gehemmt. In Gegenwart von β-Cyclodextrin war die Hemmung der Pullulanase deutlicher als die der Amylase. Siehe dazu Tabelle 2.

Tabelle 2

Enzymaktivität in Gegenwart von Cyclodextrinen

| | | | Thermoanaerobacter ethanolicus | |
|---|---|---|---|---|
| **Clostridium thermosaccharolyticum** | | | | |
| Konz. an Cyclodextrin | Amylase | Pullulanase | Amylase | Pullulanase |
| α-Cyclodex. | | | | |
| 0 mM | 100% | 100% | 100% | 100% |
| 1 mM | 115% | 125% | 119% | 107% |
| 2 mM | 117% | 106% | 112% | 95% |
| 5 mM | 118% | 111% | 114% | 116% |
| 7,5 mM | 109% | 113% | 110% | 97% |
| 10 mM | 111% | 114% | 115% | 108% |
| β-Cyclodex. | | | | |
| 0 mM | 100% | 100% | 100% | 100% |
| 1 mM | 97% | 75% | 50% | 5% |
| 2 mM | 84% | 66% | 41% | 3% |
| 5 mM | 87% | n.d. | 31% | 3% |
| 7,5 mM | 72% | 33% | 29% | 1% |
| 10 mM | 62% | 33% | 24% | 1% |

Stabilität der Enzyme

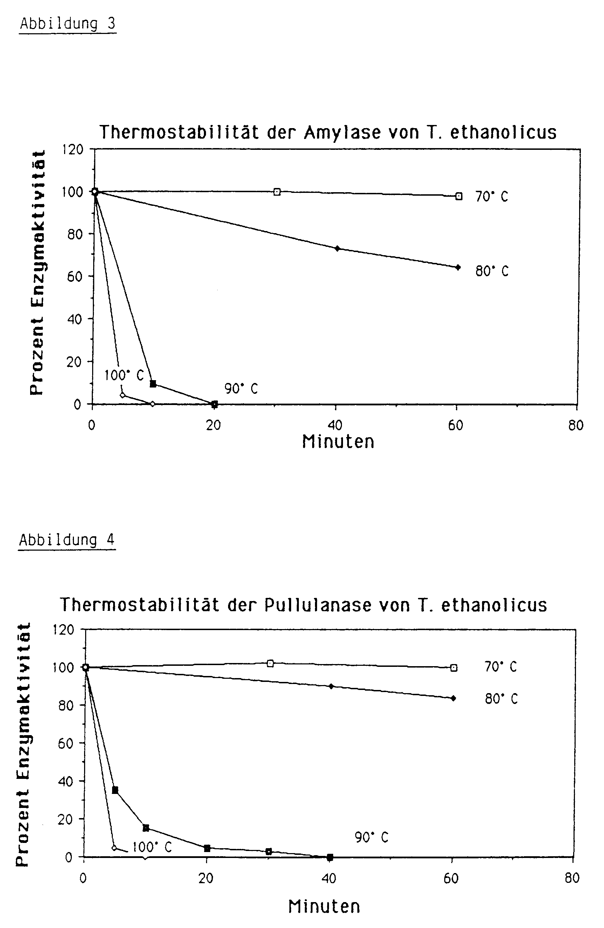

Bei pH 5,5 wurde in Abwesenheit von Substrat und ohne Zusatz von Metallionen unter aeroben Bedingungen bei 60 °C und bei 70 °C nach 80 Stunden kein Aktivitätsverlust festgestellt. Dies gilt für Amylase und Pullulanase. Die unter den gleichen Bedingungen gemessenen Stabilitäten bei 80 °C, 90 °C und 100 °C sind den Abbildungen 3 und 4 zu entnehmen.

Vergleich der Stabilität der Pullulanase mit der käuflicher Enzyme

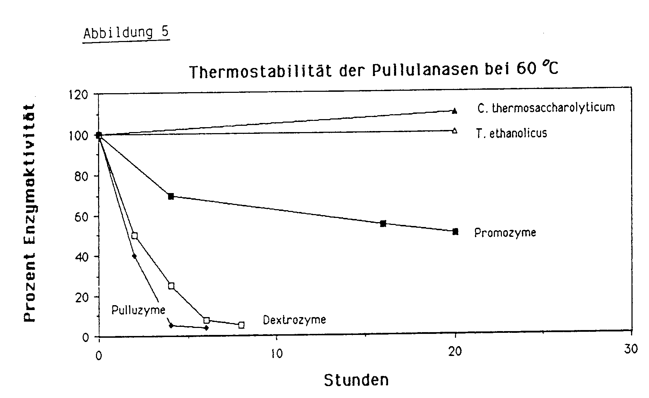

Bei 60 °C wurde die Stabilität von Promozym, Dextrozym und Pulluzym mit der der Pullulanase von Thermoanaerobacter ethanolicus verglichen. Gleiche Aktivitäten wurden eingesetzt. Siehe dazu Abbildung 5.

2) Clostridium thermosaccharolyticum DSM 572

Temperaturoptimum von Amylase und Pullulanase

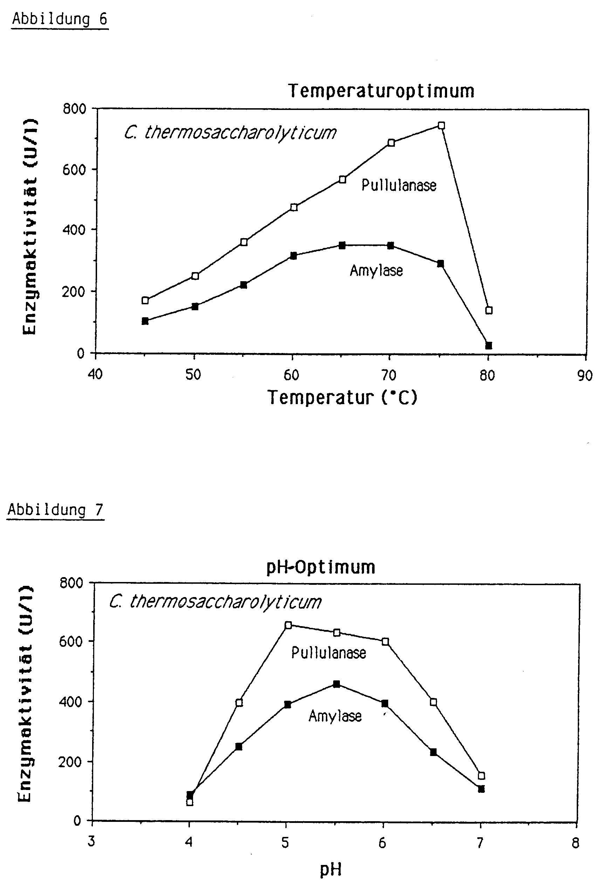

Die Amylase ist optimal aktiv zwischen 60 °C und 75 °C, insbesondere zwischen 65 °C und 70 °C. Die Pullulanase ist optimal aktiv zwischen 60 °C und 75 °C, insbesondere zwischen 65 °C und 75 °C, und ganz besonders zwischen 70 °C und 75 °C. Beide Enzyme sind aktiv bei Temperaturen zwischen 50 °C und 80 °C, insbesonders zwischen 55 °C und 78 °C, vorzugsweise zwischen 60 °C und 75 °C und besonders bevorzugt zwischen 65 °C und 75 °C. Siehe dazu Abbildung 6.

pH-Optimum von Amylase und Pullulanase

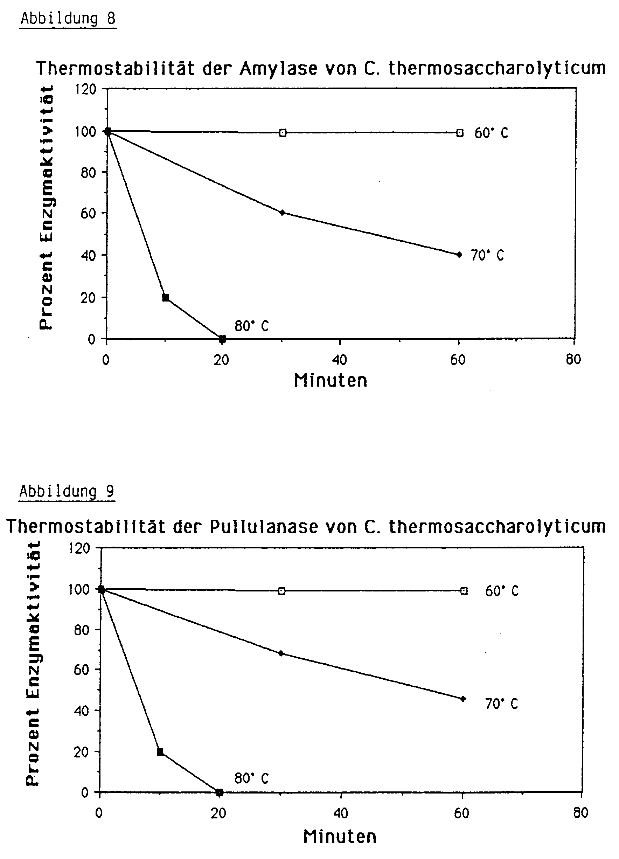

Die Amylase ist optimal aktiv zwischen pH 5,0 und 6,0. Die Pullulanase ist ebenfalls optimal aktiv zwischen pH 5,0 umd 6,0. Beide Enzyme sind aktiv bei pH-Werten zwischen 4,0 und 7,0, insbesonders zwischen pH 4,5 und 6,5 und besonders bevorzugt zwischen pH 5,0 und 6,0. Siehe dazu Abbildung 7.

Beide Enzyme sind unter den gleichen Bedingungen katalytisch aktiv, so daß sie in einem Schritt zur Stärkehydrolyse eingesetzt werden können. Hierfür werden keine Metallionen benötigt. Die Experimente wurden unter aeroben Bedingungen und ohne Substratzugabe durchgeführt.

Enzymaktivität in Gegenwart von Metallen

In Gegenwart von Zink und Kupfer fällt die Aktivität von Amylase und Pullulanase deutlich ab. Calciumionen haben sowohl auf die Aktivität der Amylase als auch auf die der Pullulanase einen stimulierenden Effekt. Siehe dazu Tabelle 1.

Enzymaktivität in Gegenwart von Cyclodextrinen

Amylase und Pullulanase werden durch $\alpha$-Cyclodextrin nicht gehemmt. $\beta$-Cyclodextrin hemmt die Aktivität der Amylase und Pullulanase leicht. Siehe dazu Tabelle 2.

Stabilität der Enzyme

Bei pH 5,3 wurde in Abwesenheit von Substrat und ohne Zusatz von Metallionen unter aeroben Bedingungen bei 60 °C nach 92 Stunden kein Aktivitätsverlust festgestellt. Dies gilt für Amylase und Pullulanase. Die unter den gleichen Bedingungen gemessenen Stabilitäten bei 70 °C und 80 °C sind den Abbildungen 8 und 9 zu entnehmen.

Vergleich der Stabilität der Pullulanase mit der käuflicher Enzyme

Bei 60 °C wurde die Stabilität von Promozym, Dextrozym und Pullucym mit der der Pullulanase von Clostridium thermosaccharolyticum verglichen. Es wurden jeweils gleiche Aktivitäten eingesetzt. Siehe dazu Abbildung 5.

Einfluß von Metallen und Cyclodextrinen

Der Einfluß von Metallen und Cyclodextrinen wurde mit konzentriertem Enzym gemessen, das zuvor gegen 20 mM Acetat-Puffer pH 5,3 (Clostridium thermosaccharolyticum) bzw. pH 5,5 (Thermoanaerobacter ethanolicus) dialysiert wurde. Es wurden Konzentrationen von 0 bis 4 mM des entsprechenden Metalls und 0 bis 10 mM des entsprechenden Cyclodextrins eingesetzt.

Die Gewinnung der erfindungsgemäßen Enzyme erfolgt gemäß dem in der europäischen Patentanmeldung 87 11 6625.2 beschriebenen Verfahren.

Danach wird die Exkretion von amylolytischen Enzymen (Amylasen und Pullulanasen) aus thermophilen, die genannten Enzyme bildenden anaeroben Bakterien in das Kulturmedium gefördert, wenn man die genannten Bakterien in einem Kohlenstoff-Quellen, Stickstoff-Quellen Mineralsalze und gegebenenfalls Vitamine enthaltenden Kulturmedium kontinuierlich kultiviert und als Kohlenstoff-Quelle eine höheres Saccharid in das Wachstum der Bakterien limitierenden Konzentrationen verwendet.

Der Ausdruck "höheres Saccharid" ist im vorliegenden Zusammenhang so zu verstehen, daß Monosaccharide ausgenommen sind. Beispiele für höhere Saccharide sind Polysaccharide, wie Stärke, Dextrin, Dextran oder Pullulan, und Oligosaccharide, wie Trisaccharide, z.B. Maltotriose, oder Disaccharide, z.B. Maltose, und deren Gemische.

Es ist dem Fachmann zuzumuten, die geeignete Konzentration des höheren Saccharids, den geeigneten pH-Wert und die geeignete Durchflußrate (Verdünnungsrate) zu ermitteln.

Lediglich zur Veranschaulichung sei angeführt, daß das Verfahren bei einer Konzentration an höherem Saccharid von 0,1 bis 3, vorzugsweise 0,2 bis 1,5 und insbesondere 0,5 bis 1,2 % (Gewicht/Volumen) durchgeführt werden kann.

Zur Veranschaulichung sei ferner ein pH-Wert von beispielsweise 4 bis 8, vorzugsweise 5,0 bis 7,5 und insbesondere 5,5 bis 7,0 genannt.

Zur Veranschaulichung sei schließlich eine Durchflußrate von beispielsweise 0,01 bis 0,4, insbesondere 0,02 bis 0,3 vorzugsweise 0,02 bis 0,2 und besonders bevorzugt von 0,03 bis 0,15 $h^{-1}$ genannt.

Hinsichtlich der Wahl einer geeigneten Temperatur wird sich der Fachmann durch richten, daß das Temperatur-Minimum durch zu langsames Zellwachstum und das Temperatur-Maximum durch Proteindenaturierung und Absterben der Zellen vorgegeben werden.

Als Kulturmedium hat sich ein Medium bewährt, das neben dem höheren Saccharid die folgenden Bestandteile enthielt (% Gewicht/Volumen): 0,25 Trypton; 0,1 Hefe-Extrakt; 0,33 $KH_2PO_4$; 0,016 $MgCl_2$; 0,0012 $CoCl_2 \cdot 6H_2O$; 0,05 Cystein·HCl. Pro l Medium wurde jeweils 1 ml einer Vitaminlösung und einer Spurenelementlösung zugegeben.

EP 0 276 806 B1

Die Kultivierung wurde in 2-l- oder 5-l-Fermentoren bei einem Kulturvolumen von 1 l bis 2 l bei 60 °C durchgeführt. Die Kulturen wurden durch einen kontinuierlichen Stickstoffstrom anaerob gehalten. Der Stickstoff wurde sterilisiert, indem man ihn durch ein sterilisiertes Baumwoll-Filter strömen ließ. Die Kulturen wurden mit 150 U/min gerührt. Der pH-Wert wurde mit einer Glaselektrode gemessen und bei dem vorgegebenen Wert gehalten, indem man automatisch 2 N KOH zugab. Die Isolierung der Enzyme erfolgte nach bekannten Techniken.

**Patentansprüche**
**Patentanspruch für folgende Vertragsstaaten : AT, BE, CH, DE, ER, GB, IT, NL**

1.  Amylolytische Enzyme, ausgewählt aus der Gruppe umfassend
Amylase aus Clostridium thermosaccharolyticum DSM 572 mit einer optimalen Aktivität bei Temperaturen zwischen 65 °C und 70 °C und bei einem pH-Wert zwischen 5,0 und 6,0,
Pullulanase aus Clostridium thermosaccharolyticum DSM 572 mit einer optimalen Aktivität bei Temperaturen zwischen 70 °C und 75 °C und bei einem pH-Wert zwischen 5,0 und 6,0,
Amylase aus Thermoanaerobacter ethanolicus DSM 2246 mit einer optimalen Aktivität bei Temperaturen zwischen 75 °C und 90 °C, insbesondere 80 °C und 90 °C und bei einem pH-Wert zwischen 5,0 und 6,0, und
Pullulanase aus Thermoanaerobacter ethanolicus DSM 2246 mit einer optimalen Aktivität bei Temperaturen zwischen 80 °C und 90 °C und bei einem pH-Wert zwischen 5,5 und 6,0.

**Patentanspruch für folgenden Vertragsstaat : ES**

1.  Verfahren zur Herstellung von amylolytischen Enzymen, ausgewählt aus der Gruppe umfassend
Amylase aus Clostridium thermosaccharolyticum DSM 572 mit einer optimalen Aktivität bei Temperaturen zwischen 65 °C und 70 °C und bei einem pH-Wert zwischen 5,0 und 6,0,
Pullulanase aus Clostridium thermosaccharolyticum DSM 572 mit einer optimalen Aktivität bei Temperaturen zwischen 70 °C und 75 °C und bei einem pH-Wert zwischen 5,0 und 6,0,
Amylase aus Thermoanaerobacter ethanolicus DSM 2246 mit einer optimalen Aktivität bei Temperaturen zwischen 75 °C und 90 °C, insbesondere 80 °C und 90 °C und bei einem pH-Wert zwischen 5,0 und 6,0, und
Pullulanase aus Thermoanaerobacter ethanolicus DSM 2246 mit einer optimalen Aktivität bei Temperaturen zwischen 80 °C und 90 °C und bei einem pH-Wert zwischen 5,5 und 6,0
durch kontinuierliches Kultivieren der genannten Bakterien in einem Kohlenstoff-Quellen, Stickstoff-Quellen, Mineralsalze und gegebenenfalls Vitamine enthaltenden Kulturmedium, wobei als Kohlenstoff-Quelle ein höheres Saccharid in das Wachstum der Bakterien limitierenden Konzentrationen verwendet wird.

**Claims**
**Claim for the following Contracting States : AT, BE, CH, DE, FR, GB, IT, NL**

1.  Amylolytic enzymes, selected from the group comprising
amylase from Clostridium thermosaccharolyticum DSM 572 with optimum activity at temperatures between 65 °C and 70 °C and at a pH between 5.0 and 6.0,
pullulanase from Clostridium thermosaccharolyticum DSM 572 with optimum activity at temperatures between 70 °C and 75 °C and at a pH between 5.0 and 6.0,
amylase from Thermoanaerobacter ethanolicus DSM 2246 with optimum activity at temperatures between 75 °C and 90 °C, particularly 80 °C and 90 °C, and at a pH between 5.0 and 6.0, and
pullulanase from Thermoanaerobacter ethanolicus DSM 2246 with optimum activity at temperatures between 80 °C and 90 °C and at a pH between 5.5 and 6.0

**Claim for the following Contracting State : ES**

1.  Process for the preparation of amylolytic enzymes, selected from the group comprising
amylase from Clostridium thermosaccharolyticum DSM 572 with optimum activity at temperatures between 65 °C and 70 °C and at a pH between 5.0 and 6.0,
pullulanase from Clostridium thermosaccharolyticum DSM 572 with optimum activity at temperatures between 70 °C and 75 °C and at a pH between 5.0 and 6.0,

7

amylase from Thermoanaerobacter ethanolicus DSM 2246 with optimum activity at temperatures between 75 °C and 90 °C, particularly 80 °C and 90 °C, and at a pH between 5.0 and 6.0, and

pullulanase from Thermoanaerobacter ethanolicus DSM 2246 with optimum activity at temperatures between 80 °C and 90 °C and at a pH between 5.5 and 6.0

by continuously cultivating the aforementioned bacteria in a culture medium containing carbon sources, nitrogen sources, mineral salts and, if necessary, vitamins, a higher saccharide being used as the carbon source in concentrations limiting bacterial growth.

**Revendications**

**Revendication pour les Etats contractants suivants : AT, BE, CH, DE, FR, GB, IT, NL**

1. Enzymes amylolytiques, sélectionnés dans le groupe comprenant

l'amylase de Clostridium thermosaccarolyticum DSM 572 ayant une activité optimale à des températures comprises entre 65 °C et 70 °C et à un pH compris entre 5,0 et 6,0

la pullulanase de Clostridium thermosaccharolyticum DSM 572 ayant une activité optimale à des températures comprises entre 70 °C et 75 °C et à un pH compris entre 5,0 et 6,0

l'amylase de Thermoanaerobacter ethanolicus DSM 2246 ayant une activité optimale à des températures comprises entre 75 °C et 90 °C et à un pH compris entre 5,0 et 6,0 et

la pullulanase de Thermoanaerobacter ethanolicus DSM 2246 ayant une activité optimale à des températures comprises entre 80 °C et 90 °C et à un pH compris entre 5,5 et 6,0.

**Revendication pour l'Etat contractant suivant : ES**

1. Procédé de préparation d'enzymes amylolytiques, sélectionnés dans le groupe comprenant

l'amylase de Clostridium thermosaccharolyticum DSM 572 ayant une activité optimale à des températures comprises entre 65 °C et 70 °C et à un pH compris entre 5,0 et 6,0

la pullulanase de Clostridium thermosaccharolyticum DSM 572 ayant une activité optimale à des températures comprises entre 70 °C et 75 °C et à un pH compris entre 5,0 et 6,0

l'amylase de Thermoanaerobacter ethanolicus DSM 2246 ayant une activité optimale à des températures comprises entre 75 °C et 90 °C et à un pH compris entre 5,0 et 6,0 et

la pullulanase de Thermoanaerobacter ethanolicus DSM 2246 ayant une activité optimale à des températures comprises entre 80 °C et 90 °C et à un pH compris entre 5,5 et 6,0

par culture continue des bactéries susmentionnées dans un milieu contenant des sources carbonées, azotées, sels minéralisés et éventuellement vitaminées, la source carbonée utilisée étant un saccharide complexe à des concentrations limitant la croissance des bactéries.

Abbildung 1

## Temperaturoptimum

Abbildung 2

## pH Optimum

Abbildung 3

Thermostabilität der Amylase von T. ethanolicus

Abbildung 4

Thermostabilität der Pullulanase von T. ethanolicus

Abbildung 5

## Thermostabilität der Pullulanasen bei 60 °C

Abbildung 6

## Temperaturoptimum

*C. thermosaccharolyticum*

Pullulanase

Amylase

Enzymaktivität (U/l)

Temperatur (°C)

Abbildung 7

## pH-Optimum

*C. thermosaccharolyticum*

Pullulanase

Amylase

Enzymaktivität (U/l)

pH

EP 0 276 806 B1

Abbildung 8

## Thermostabilität der Amylase von C. thermosaccharolyticum

Abbildung 9

## Thermostabilität der Pullulanase von C. thermosaccharolyticum

13